# EUROPEAN PATENT SPECIFICATION

(11) **EP 4 623 849 B1**
(45) Date of publication and mention of the grant of the patent: **02.12.2026**
(21) Application number: 25165465.3
(22) Date of filing: 21.03.2025
(51) Int. Cl.: A61B 34/10, A61B 34/20, A61B 34/00, A61B 90/30, A61B 90/00

(54) **SURGICAL GUIDANCE USING A STRUCTURED LIGHT CAMERA**
CHIRURGISCHE FÜHRUNG MIT EINER STRUKTURIERTEN LICHTKAMERA
GUIDAGE CHIRURGICAL UTILISANT UNE CAMÉRA À LUMIÈRE STRUCTURÉE

(30) Priority: 25.03.2024 US 202463569357 P
(43) Date of publication of application: 01.10.2025
(73) Proprietor: Orthosoft ULC, Montreal , QC H3C 2N6 (CA)
(72) Inventor: MADIER VIGNEUX, Joseph, Montreal, H1X 3A3 (CA); MULLEMEESTER, Mélanie, 34130 Mauguio (FR); TWYFORD, Perry T., Brecksville, 44141 (US); SAUVEE, Mickael, 34130 Montpellier (FR)
(74) Representative: Taor, Simon Edward William

(56) References cited:
- WO-A2-2023/086592
- US-A1- 2019 076 195

## Description

### CLAIM OF PRIORITY

This application claims the benefit of U.S. Provisional Patent Application Serial No. 63/569,357, filed on March 25, 2024, the benefit of priority of which is claimed hereby.

### TECHNICAL FIELD

Examples described herein generally relate to a structured light camera and, more specifically, to surgical guidance using a structured light camera.

### BACKGROUND

Surgical guidance systems are an integral part of modern surgical practice, providing critical support to surgeons by enhancing visualization, precision, and control during complex procedures. These systems encompass a range of technologies that work in concert to facilitate the planning and execution of surgeries, particularly those requiring high levels of accuracy due to the sensitive nature of the anatomical structures involved. US2019076195 discloses that a surgical plan is created within the image data set. An anatomical region is registered to the surgical plan. A laser is articulated to project a light indication on the subject indicative of a depth of a first incision. A system for implementing the surgical plan on a subject includes a tracking array. A processor receives an initial positional input from the tracking array, three-dimensional scan data of a surgical field and includes software for generating a surgical plan. A laser is positioned to project a light indication on the subject. A controller controls the laser to modify the light indications to indicate a preselected path and a preselected depth of incision. The articulating device receives the position input and the surgical plan.

### SUMMARY

In examples, a system for projecting surgical instructions upon a patient during a surgical procedure is provided as set out in claim 1. Additional features are set out in claims 2 to 10.

### BRIEF DESCRIPTION OF THE DRAWINGS

Various examples are illustrated in the figures of the accompanying drawings. Such examples are demonstrative and not intended to be exhaustive or exclusive examples of the present subject matter.
FIG. 1 illustrates a schematic diagram of an example system for surgical guidance using a structured light camera.
FIG. 2 is a block diagram of an example of a system for surgical guidance using a structured light camera, according to an embodiment.
FIG. 3 illustrates a method 300 for providing surgical guidance during a surgical procedure.
FIG. 4 illustrates a schematic diagram of a projected shape on a head of a patient during a surgical procedure.
FIG. 5 illustrates a method for providing surgical guidance during a surgical procedure.
FIG. 6 illustrates a schematic diagram showing an example three-dimensional model and an example image captured during a surgical procedure.
FIG. 7 illustrates an example alignment of an example three-dimensional model and an example image captured during a surgical procedure.
FIG. 8 illustrates a method 800 for providing surgical guidance during a surgical procedure.
FIG. 9 illustrates a schematic diagram showing an example three-dimensional model of a tool during a surgical procedure.
FIG. 10 illustrates a method 1000 for providing surgical guidance during a surgical procedure.
FIG. 11 illustrates an example heat map of the brain projected on a patient during a surgical procedure.
FIG. 12 illustrates a method 1200 for providing surgical guidance during a surgical procedure.
FIG. 13 illustrates an example brain atlas projected on a patient during a surgical procedure.
FIG. 14 is a block diagram illustrating an example of a machine upon which one or more examples may be implemented.

### DETAILED DESCRIPTION

There are many challenges in the realm of surgical guidance during surgical procedures, particularly for surgical procedures involving intricate and delicate operations such as neurosurgery. One of the primary problems in such procedures is the difficulty in accurately translating pre-operative planning into the intraoperative phase. Surgeons must navigate complex anatomical structures and make precise incisions and interventions, often relying on static imaging data that may not reflect real-time changes in the surgical field. Additionally, maintaining spatial awareness of critical structures and safe zones within the target area is paramount to avoid damaging vital tissues, which can lead to significant postoperative complications.

This disclosure relates to a sophisticated surgical guidance system that can integrate advanced imaging, real-time structured light projection, and computational processing to provide dynamic, interactive assistance during surgery. The system can include a three-dimensional camera, a structured light projector, and a computation module that can process pre-operative medical imaging data to generate a detailed three-dimensional model of the target area. This model can be used to create an adjusted visual representation of the surgical instructions, which can be projected onto the patient's body. The projection can be configured to adapt in real-time to movements and changes in the patient's anatomy, ensuring that the guidance remains accurate throughout the surgical procedure.

The system described herein can enhance the surgeon's ability to visualize the surgical plan directly on the patient's body, thereby improving the accuracy of incisions and the placement of surgical tools. The system can also provide real-time updates and adjustments to the projected guidance based on intraoperative imaging, which can provide a dynamic and responsive approach to surgery. The system's ability to project critical information such as entry points, safety zones, tool models, heat maps, and anatomical atlases directly onto the surgical field can help increase the surgeon's spatial awareness and reduce the risk of inadvertent damage to critical structures. By addressing these challenges, the present disclosure significantly contributes to the safety, efficiency, and success of surgical procedures.

The above discussion is intended to provide an overview of the subject matter of the present patent application. It is not intended to provide an exclusive or exhaustive explanation of the invention. The description below is included to provide further information about the present patent application.

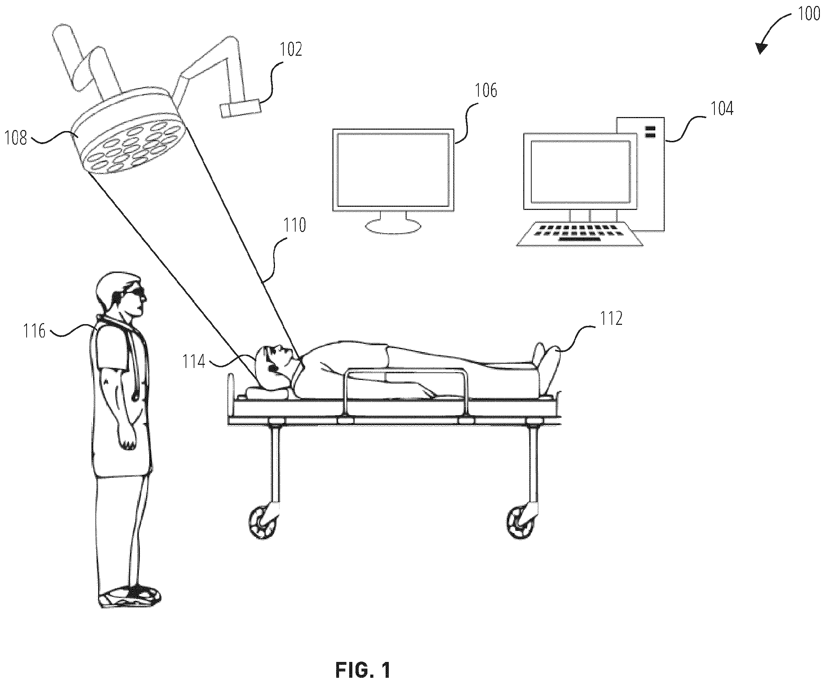

FIG. 1 illustrates a schematic diagram of an example system 100 for surgical guidance using a structured light camera. The system 100 can be configured to provide step-by-step instructions to a medical professional during a surgical procedure. The system 100 can project planned surgical steps upon a target area for surgical procedure 114 to help aid the medical professional during the surgical procedure. The system 100 can also provide medical information (e.g., insertion points, insertion angles, heat maps to show distances between the planned insertion points, brain atlas to show anatomical features of the brain, or the like). The system 100 can include a three-dimensional camera 102, a computation module 104 (e.g., computer, integrated computing device, or the like), a display 106, and a structured light projector 108. The system 100 can be configured to project a projected structured light 110 onto a target area for surgical procedure 114 of a patient 112 to provide guidance during the surgical procedure to a medical professional 116.

The three-dimensional camera 102 can be configured to capture three-dimensional images of objects (e.g., the target area for surgical procedure 114 of the patient) within a field of view of the three-dimensional camera 102. The three-dimensional camera 102 can be in communication with the computation module 104, the display 106, or any other component of the system 100. The three-dimensional camera 102 can include multiple cameras positioned in varying orientations around the environment of the surgical procedure. In an example, a Zivid 2 M70 structured light camera (from Zivid in Oslo Norway) can be used as the three-dimensional camera 102. In this example, the Zivid 2 M70 includes an integrated structure light projector, such as structure light projector 108. Accordingly, the Zivid 2 M70 integrates the three-dimensional camera 102 and the structured light projector 108 into a single device. For the purposed of this disclosure, the three-dimensional camera 102 and structured light projector 108 are discussed as separate entities. The projection of surgical procedure guidance information is a modification to the standard structured light projection used by a structured light three-dimensional camera such as the Zivid 2 M70.

The computation module 104 can be configured to receive the images captured by the three-dimensional camera 102 and perform one or more operations based on the information gathered from the medical images captured by the three-dimensional camera 102. The computation module 104 can be configured to change the operating parameters of any of the components of the system 100. In examples, the computation module 104 can receive a signal from the three-dimensional camera 102 and use that signal to provide context to information gathered by other components of the system 100 (e.g., the structured light projector 108). The computation module 104 can generate outputs that can be projected onto the target area for surgical procedure 114 of the patient 112 via the structured light projector 108.

The display 106 can be configured to communicate information between the system 100 and the 116. In examples, the display 106 can show the medical images captured by the three-dimensional camera 102, or objects that will be projected by the structured light projector 108. As such, the display 106 can be configured to duplicate the information projected by the structured light projector 108 or to provide information that supplements the information projected by the structured light projector 108. The display 106 can also include a user interface integrated with the display 106 or as a separate component in communication with the display 106. The user interface can allow the medical professional 116 to interact with the system 100, customize the visual aids, and access additional medical information as needed. This interface can support touch input, voice commands, or other input methods to ensure that the medical professional can interact with the system 100 without compromising sterility during the surgical procedure.

The structured light projector 108 can be configured to project intricate patterns of light onto the target area for surgical procedure 114 of the patient 112. These patterns, known as projected structured light 110, can be used to create detailed visual aids that can include, but are not limited to, insertion points, insertion angles, heat maps, and anatomical features such as a brain atlas. The structured light projector 108 can operate in conjunction with the three-dimensional camera 102 and the computation module 104 to adjust the projected visual aids in real-time, ensuring accuracy and relevance to the ongoing surgical procedure. This dynamic adjustment capability can allow the system 100 to adapt to changes in the surgical field or in the positioning of the patient 112.

The structured light projector 108 can project visual aids that are not only limited to static images but can also include dynamic, interactive elements. According to the invention, it can project a live heat map that changes as the medical professional 116 moves surgical tools within the target area, providing immediate visual feedback on tool positioning relative to critical structures. Similarly, the structured light projector 108 can update the brain atlas projection in real-time to reflect the current phase of the surgical procedure, highlighting areas of interest or caution as the surgery progresses.

The structured light projector 108, in collaboration with the computation module 104, can utilize advanced algorithms (e.g., instructions) to generate a three-dimensional representation of the surgical field. This representation can be projected directly onto the patient 112, allowing the medical professional 116 to see beneath the surface of the target area without making an incision. The projection of the three-dimensional representation can significantly enhance the precision of the surgical procedure, reduce the risk of complications, and improve patient outcomes.

Additionally, the system 100 can be equipped with machine learning capabilities, enabling it to learn from each surgical procedure and improve its performance over time. For example, the system can analyze the outcomes of previous surgeries to refine the accuracy of its projections, adapt its algorithms for generating visual aids, and even suggest optimal surgical approaches based on historical data.

In summary, the system 100 for surgical guidance using the three-dimensional camera 102 and the computation module 104 to provide real-time visual aids directly on the patient's body can improve the predictability of surgical procedures. The system 100 can enhance the precision and safety of surgical procedures, support the decision-making process of the medical professional 116, and ultimately contribute to improved patient care.

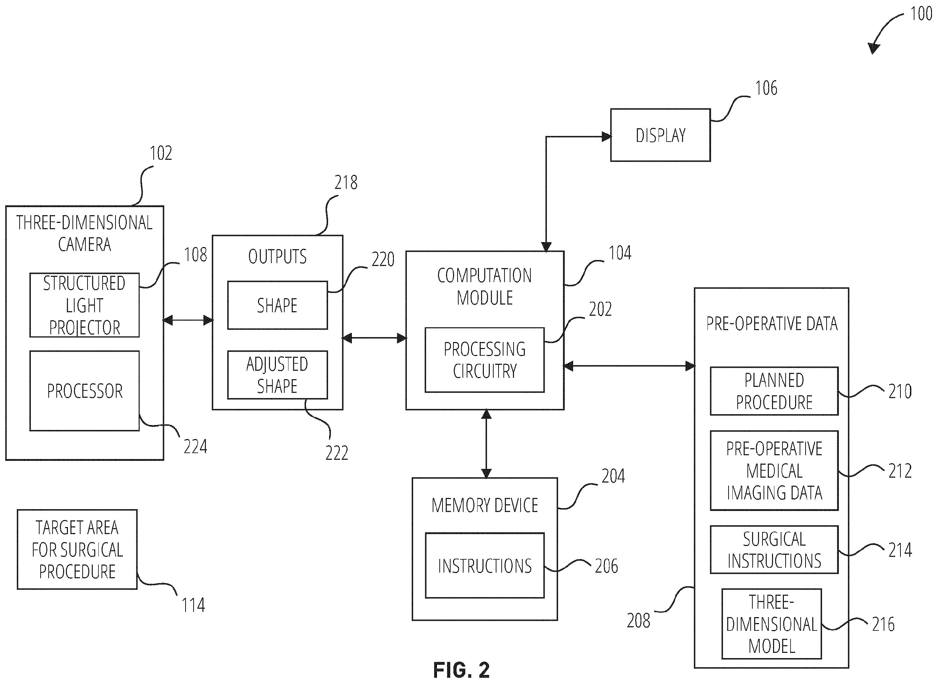

FIG. 2 is a block diagram of an example of the system 100 for surgical guidance using a structured light camera, according to an embodiment.

The computation module 104 can include processing circuitry 202 that can be communicatively coupled to a memory device 204 including instructions 206. The instructions 206, when initiated by the processing circuitry 202 of the computation module 104, can be configured to cause the processing circuitry 202 to communicate, control, or otherwise interact with at least one component of the system 100. The computation module 104 can access a database including pre-operative data 208 to before, during, or after a surgical procedure. The computation module 104 can use the pre-operative data 208 to inform or supplement any of the operations or generated information computed by the computation module 104 during the surgical procedure.

The pre-operative data 208 can be obtained before the surgical procedure or in any step that occurs within the surgical procedure that predates the current step or operation of the surgical procedure. In examples, the pre-operative data 208 can include pre-operative medical imaging data 212, planned procedure 210, a surgical instructions 214, a three-dimensional model 216, or the like.

The planned procedure 210 can include step-by-step instructions for the surgical procedure as planned. The planned procedure 210 can be generated preoperatively based on the pre-operative medical imaging data 212, the three-dimensional model 216, or other medical information of the patient. The planned procedure 210 can include techniques and instruments that are planned to be used for the surgical procedure. For example, the 210 can include details of the sequence of techniques, the techniques to be employed, the instruments required, or the like. This planned procedure 210 can be prepared based on the pre-operative data 208, ensuring that every aspect of the surgery is carefully considered and planned for optimal outcomes of the surgical procedure.

The pre-operative medical imaging data 212 can be medical imaging captured using imaging equipment (e.g., X-ray, computed tomography (CT), MRI, ultrasound, positron emission tomography, bone densitometry, or the like) that includes relevant portions of the patient for the surgical procedure. For example, the pre-operative medical imaging data 212 can include medical images of the target area for surgical procedure 114. The pre-operative medical imaging data 212 can provide a multidimensional view of the patient's anatomy, enabling the surgical team to plan and execute procedures with an unprecedented level of precision and confidence. The pre-operative medical imaging data 212 can help identify the surgical target, understand complex relationships between different anatomical structures, and ensure that the surgical approach is both effective and minimally invasive.

The surgical instructions 214 can be a guide for the medical professional 116, detailing specific actions, techniques, and considerations necessary for the successful execution of the surgical procedure. The surgical instructions 214 can be derived from at least one of the pre-operative data 208, including the pre-operative medical imaging data 212, the planned procedure 210, and the three-dimensional model 216.

The three-dimensional model 216 can include a digital reconstruction of the patient's anatomy relevant to the surgical procedure. The three-dimensional model 216 can be generated from the pre-operative medical imaging data 212 or medical images captured during the surgical procedure. The three-dimensional model 216 can provide a comprehensive and interactive representation of the patient's anatomical structures, allowing for detailed pre-operative planning and simulation of the surgical procedure. By incorporating the three-dimensional model 216 into the pre-operative planning process and utilizing it throughout the surgical procedure, the surgical team can achieve a higher level of precision and confidence.

As shown in FIG. 2, the three-dimensional camera 102 can include the structured light projector 108 and a processor 224. As such, the three-dimensional camera 102 can capture medical images, process the captured medical images, and project the projected structured light 110 onto the patient. The processor 224 can be in communication with the processing circuitry 202 to transfer captured medical images or receive signals to project within the projected structured light 110.

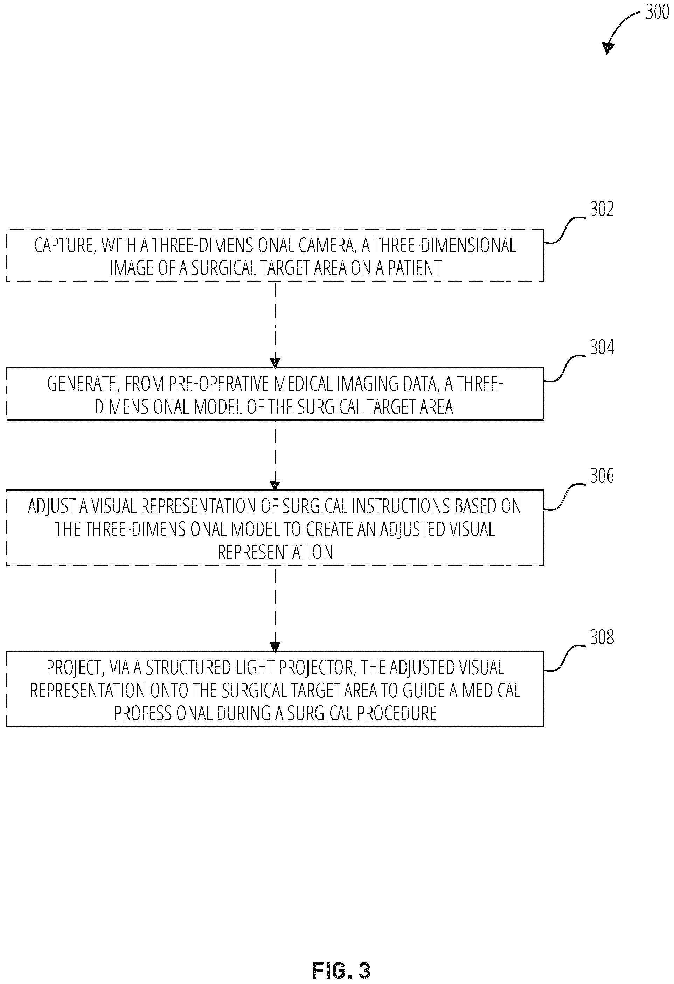

FIG. 3 illustrates a method 300 for providing surgical guidance during a surgical procedure. Although the example method 300 depicts a particular sequence of operations. The sequence may be altered. For example, some of the operations depicted may be performed in parallel or in a different sequence that does not materially affect the function of the method 300. In other examples, different components of an example device or system that implements the method 300 can perform functions at substantially the same time or in a specific sequence. As shown in FIG. 3, the method 300 can include at least one of operation 302 - operation 308.

At operation 302, the method 300 can optionally include capturing, with a three-dimensional camera (e.g., three-dimensional camera 102 (FIG. 1)) a three-dimensional image of a surgical target area (e.g., the target area for surgical procedure 114 (FIG. 1)) of a patient (e.g., patient 112 (FIG. 1)). The captured medical image can be transmitted to the computation module 104 (FIG. 1), or more specifically, to the processing circuitry 202 (FIG. 2), for further processing by the system 100. In this operation, the three-dimensional camera 102 can utilize the structured light component 108 in capturing the three-dimensional image of the surgical target area.

At operation 304, the method 300 can optionally include generating, from the pre-operative medical imaging data (e.g., the pre-operative medical imaging data 212 (FIG. 2)), a three-dimensional model (e.g., the three-dimensional model 216 (FIG. 2)) of the surgical target area. The three-dimensional model can be used by any component of the system 100 to generate or analyze information to further benefit the medical professional throughout the surgical procedure.

At operation 306, method 300 can optionally include adjusting a visual representation of surgical instructions (e.g., the surgical instructions 214 (FIG. 2)) based on the three-dimensional model to create an adjusted visual representation. Adjustments to the visual representation can include a variety of modifications that enhance the clarity, accuracy, and usefulness of the surgical guidance provided to the medical professional. These adjustments can be made possible by the dynamic nature of the three-dimensional model and the computational power of the system 100, which together allow for real-time updates and refinements to the visual aids. For example, the adjustments to the visual representation can include moving the projected location of the visual representation based on the movement of the patient during the surgical procedure, movement of the target area for the surgical procedure during the surgical procedure, or variations occurring from the planned procedures during the surgical procedure. Adjustments can also include scaling or skewing shapes to ensure the procedural guidance is presented to the medical professional as the intended shape or outline and accounts for irregularities of the projection surface (e.g., patient's skull or other anatomy).

At operation 308, the method 300 can optionally include projecting, via a structure light projector (e.g., structured light projector 108 (FIG. 1)), the adjusted visual representation (e.g., from operation 306) onto the surgical target area to guide the medical professional during the surgical procedure. This projection (e.g., projected structured light 110 (FIG. 1) can provide a real-time navigational aid, overlaying critical information (e.g., anatomical information, personal medical information of the patient, planned surgical procedures, planned surgical trajectories, or the like) directly onto the patient's body or the surgical field. The structured light projector (e.g., the structured light projector 108) can provide precise and clear visualization of the supplemental information, which can be helpful to the medical professional during the surgical procedure. As discussed herein, utilizing the structured light component of the three-dimensional camera provides the benefit of projecting surgical guidance directly onto the surgical field without additional components or systems further complicating the surgical environment.

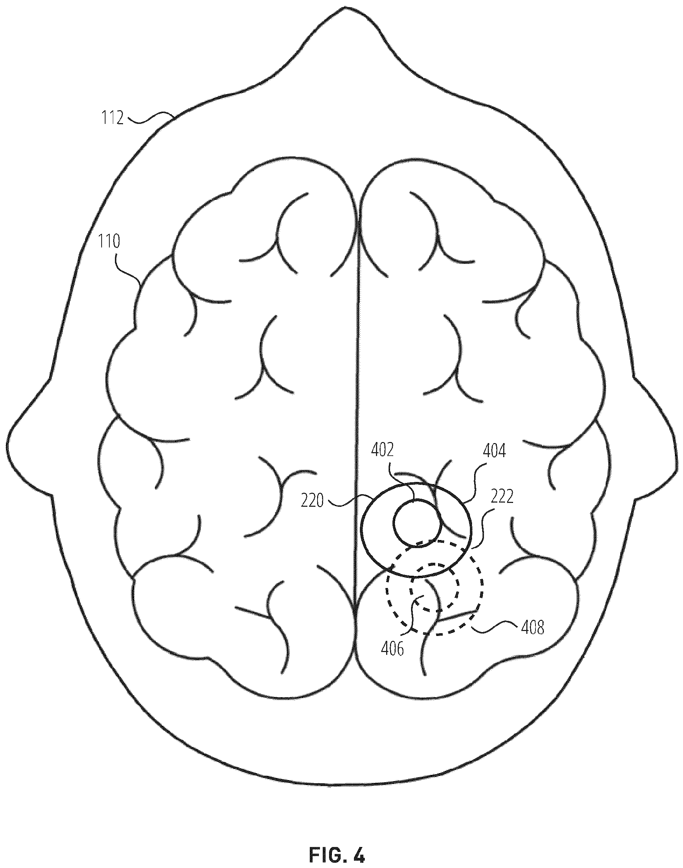

FIG. 4 illustrates a schematic diagram of a projected structured light (e.g., the projected structured light 110 (FIG. 1)) on a target area (e.g., the target area for surgical procedure 114) of a patient (e.g., patient 112) during a surgical procedure. As shown in FIG. 4, the projected structured light 110 can include a shape 220 and an adjusted shape 222. The shape 220 can include a planned entry point 402 and a safety zone 404. The adjusted shape 222 can include an updated planned entry point 406 and an updated safety zone 408. As shown in FIG. 4, the entry points and the updated safety zones can include circular, oval, triangular, square, rectangular, or any other shape that can indicate a planned entry point or safety zone. As the surgical procedure progresses, the initial projection (shape 220) can be updated to reflect changes in the surgical plan or to accommodate for shifts in the patient's anatomy, such as movement or tissue deformation. The adjusted shape (e.g., adjusted shape 222) can represent such updates to the shape provided to the medical professional in the projected structured light 110.

As shown in FIG. 4, the system 100 can improve the placement of the shape (e.g., from the shape 220 to the adjusted shape 222) intraoperatively based on information captured by the three-dimensional camera (e.g., the three-dimensional camera 102 (FIG. 1)) to improve the precision of the information in the projected structured light 110 (FIG. 1). The diagram illustrates the transition from the initially planned projection (shape 220) to an adjusted projection (adjusted shape 222) that takes into account real-time information and adjustments made during the surgery.

The planned entry point 402 can be a visual marker projected (e.g., via the projected structured light 110 (FIG. 1)) onto the patient's body, indicating a precise location where a surgical incision or entry is initially planned based on pre-operative data. The planned entry point 402 can be a guide for the medical professional to initiate the procedure at the correct anatomical site.

The safety zone 404 can surround the planned entry point 402. The safety zone 404 can delineate areas that should be avoided during the surgical procedure. In examples, the safety zone 404 can represent areas that need to be prepped (e.g., shaved, cleaned, or otherwise prepared) before the medical professional can begin the surgical procedure.

The updated planned entry point 406 can be a revised location for the surgical entry, which has been adjusted based on intraoperative findings or changes in the surgical plan. The updated planned entry point 406 can ensure that the entry point remains accurate and relevant throughout the procedure.

The updated safety zone 408 can reflect changes to the boundaries surrounding the updated planned entry point 406 during the surgical procedure. In examples, the updated safety zone 408 can be expanded, contracted, shifted, or altered in any other way to ensure ongoing indication of important surgical considerations.

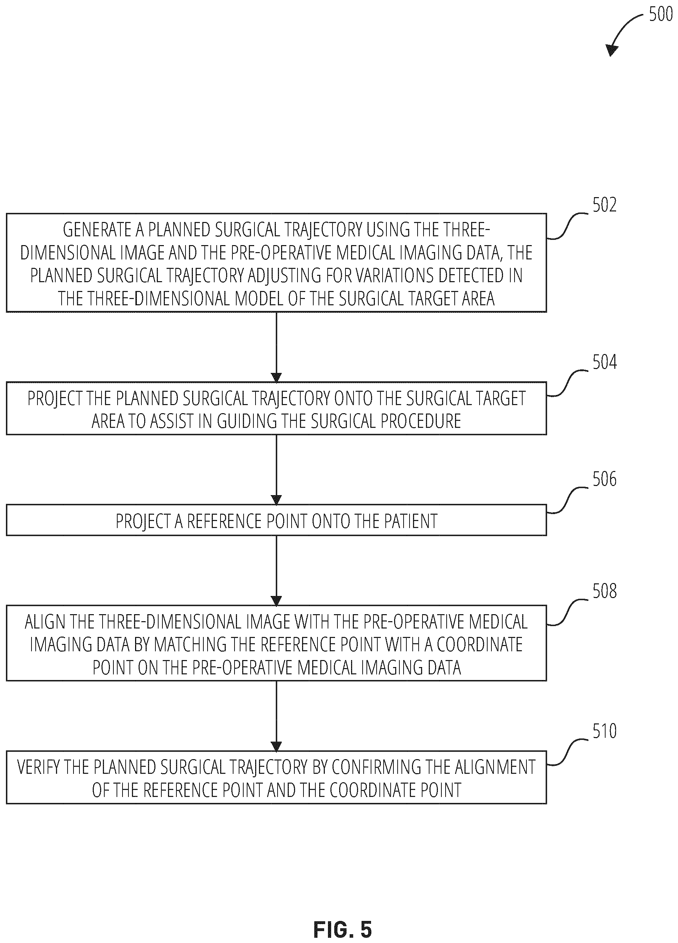

FIG. 5 illustrates a method 500 for providing surgical guidance during a surgical procedure. Although the example method 500 depicts a particular sequence of operations, the sequence may be altered . For example, some of the operations depicted may be performed in parallel or in a different sequence that does not materially affect the function of the method 500. In other examples, different components of an example device or system that implements the method 500 can perform functions at substantially the same time or in a specific sequence. As shown in FIG. 5, the method 500 can include at least one of operation 502 - operation 510.

At operation 502, method 500 can optionally include generating a planned surgical trajectory (e.g., the adjusted shape 222) using the three-dimensional image and the pre-operative medical imaging data. The planned surgical trajectory can adjust for variations detected in the three-dimensional model of the surgical target area. At operation 504, the method 500 can include projecting the planned surgical trajectory onto the surgical target area (e.g., via the projected structured lights 110 (FIG. 1)) to assist in guiding the surgical procedure.

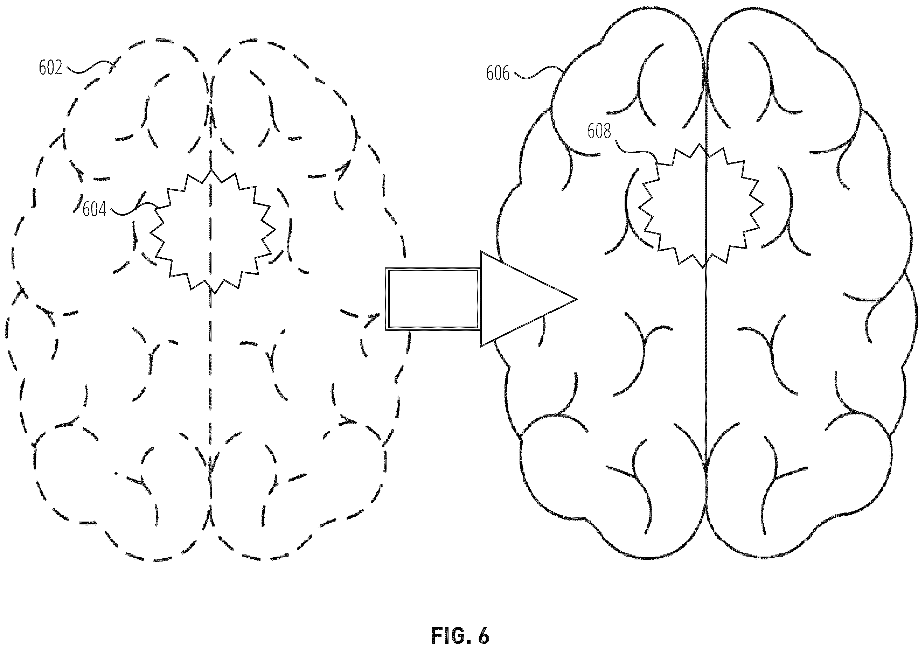

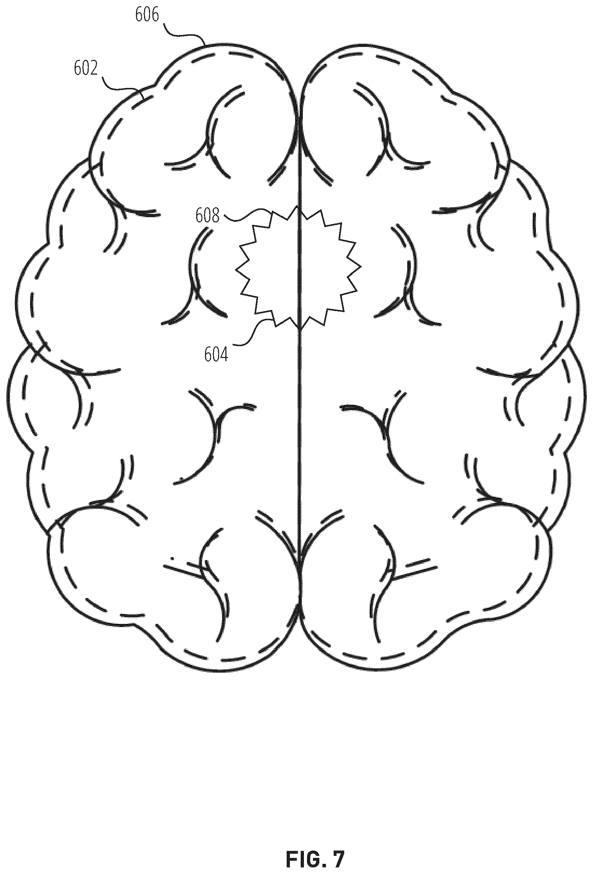

At operation 506, the method 500 can optionally include projecting a reference point onto the patient. At operation 508, the method 500 can include aligning the three-dimensional image with the pre-operative medical imaging data by matching the reference point with a coordinate point on the pre-operative medical imaging data. In operation 510, method 500 verifies the planned surgical trajectory by confirming the alignment of the reference point and the coordinate point. Operation 502 - operation 510 are graphically represented in FIG. 6 and FIG. 7 below.

FIG. 6 illustrates a schematic diagram showing an example three-dimensional model (e.g., pre-operative medical imaging data 602) and an example image (e.g., three-dimensional image 606) captured during a surgical procedure.

As discussed in FIG. 5, operation 504 of the method 500 can include the processing circuitry (e.g., the processing circuitry 202 (FIG. 2) projecting (e.g., via the projected structured light 110 (FIG. 1)) the pre-operative medical imaging data 602 including the coordinate point 604 onto the patient. Further, operation 506 of the method 500 can include the processing circuitry 202 (FIG. 2) projecting (e.g., via the projected structured light 110 (FIG. 1)) a reference point 608 onto the patient (e.g., as shown projected with the three-dimensional image 606 in FIG. 6).

The coordinate point 604 and the reference point 608 can help the computation module (e.g., the processing circuitry 202 of the computation module 104) align the pre-operative medical imaging data 602 to the three-dimensional image 606 on the patient (e.g., the patient 112 (FIG. 1).

FIG. 7 illustrates a schematic diagram showing the alignment of the pre-operative medical imaging data 602 and the three-dimensional image 606. As discussed herein, the pre-operative medical imaging data 602 and the three-dimensional image 606 can be aligned by the computation module 104 or the processing circuitry 202 by aligning the coordinate point 604 and the reference point 608, as discussed with reference to FIG. 5 relative to operation 508 of the method 500.

Overall, method 500 (shown in FIG. 5) and the graphical representations of method 500 (shown in FIG. 6 and FIG. 7) highlight a process for integrating pre-operative planning with intraoperative guidance. The method 500 can leverage advanced imaging and projection technologies to create a seamless and dynamic surgical guidance system. By aligning pre-operative medical imaging data with real-time three-dimensional images, the system can provide the medical professional with accurate and actionable information, which can enhance the precision of the surgical procedure and potentially improve patient outcomes.

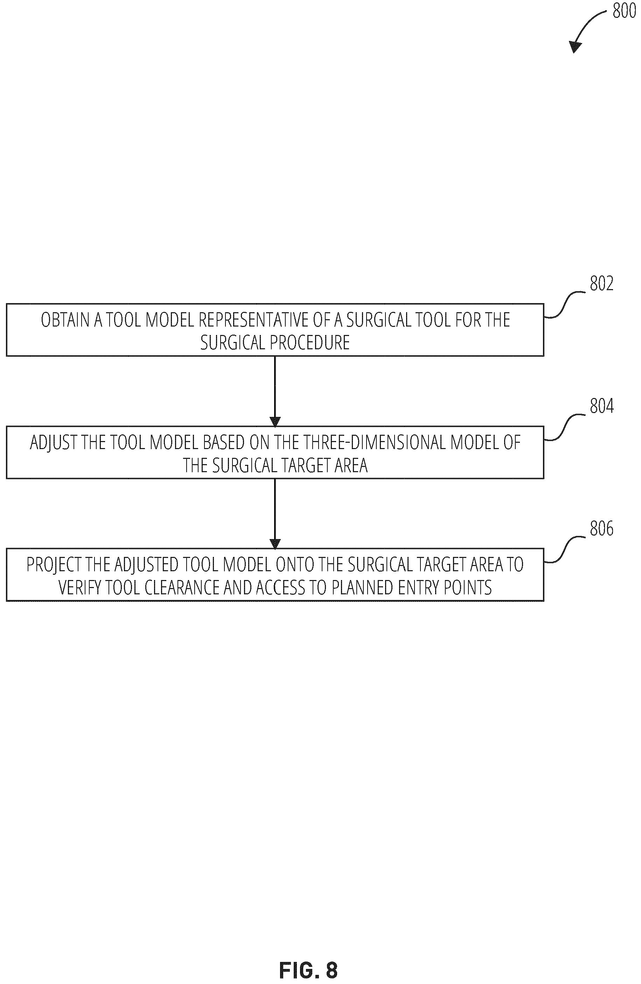

FIG. 8 illustrates a method 800 for providing surgical guidance during a surgical procedure. In examples, the method 800 can obtain and project a model of a tool onto a target area for the surgical procedure to help the medical professional visualize the placement of tools during the surgical procedure. Although the example method 800 depicts a particular sequence of operations, the sequence may be altered . For example, some of the operations depicted can be performed in parallel or in a different sequence that does not materially affect the function of the method 800. In other examples, different components of an example device or system that implements the method 800 can perform functions at substantially the same time or in a specific sequence. The method 800 can include at least one of operation 802 - operation 806.

At operation 802, the method 800 can optionally include obtaining a tool model representative of a surgical tool for the surgical procedure. Operation 802 can be foundational for integrating the use of surgical tools into the overall surgical plan and for ensuring that the tools are appropriate for the specific procedure being performed. Moreover, the operation 802 can help ensure that the medical professional uses the appropriate tools for the corresponding step or procedure of the surgical procedure. The tools can be selected by choosing tools that are best suited for the type of surgery, the current step or process within the planned surgery, and the patient's specific anatomy. Once the tools are selected, the computation module 104 (FIG. 1) can generate or obtain digital models from a pre-existing database.

At operation 804, the method 800 can optionally include adjusting the tool model based on the three-dimensional model of the surgical target area. Operation 804 can ensure that the tool model is tailored to the patient's anatomy as it will be encountered during the surgery. For example, the size, extension, or bits of the tools can be adjusted based on medical information from the three-dimensional model. The adjustments to the tool can also ensure that the tool will be able to reach the target area (e.g., the target area for surgical procedure 114 (FIG. 1)) without obstruction from another tool, the anatomy of the patient, or the like. The adjusted tool model can be used to simulate the surgical procedure. Such a simulation of the use of the adjusted tool model can simulate the actual surgical conditions of the tool being used on the patient, which can allow the surgical team to plan the use of the tool in a virtual environment that mimics the actual surgical conditions. The adjustments to the tool can also help the surgical team verify that the size and shape of the tool are appropriate for the surgical target area, which can ensure the tool can be used without causing unplanned contact between the tool and the patient. The adjusted tool can also be optimized for the specific surgical procedure on that patient. For example, the adjusted tool can include angle of entry, depth of penetration, and set thresholds for inputs, or variables of the control inputs of the planned tool, or the like.

At operation 806, the method 800 can optionally include projecting the adjusted tool model onto the surgical target area to verify tool clearance and access to planned entry points. The project can provide a final verification for the medical professionals before they begin the surgical procedure (or that step or task of the surgical procedure). Such a projection of the tool onto the target area of the surgical procedure can provide verification of clearance between the tool and the anatomy of the patient and other tools used in the surgical procedure, confirm the planned access points are accessible with the planned tool and that the tool can be maneuvered as intended within the constraints of the surgical site. The projection of the adjusted tool can also provide intraoperative guidance to the surgical team. The intraoperative guidance can be provided in real-time to ensure that the tool is used according to the surgical plan. The projection of the adjusted tool can also improve the precision of the use of the tool by the surgical team, which can decrease the risk of errors and improve overall outcomes of the surgical procedure.

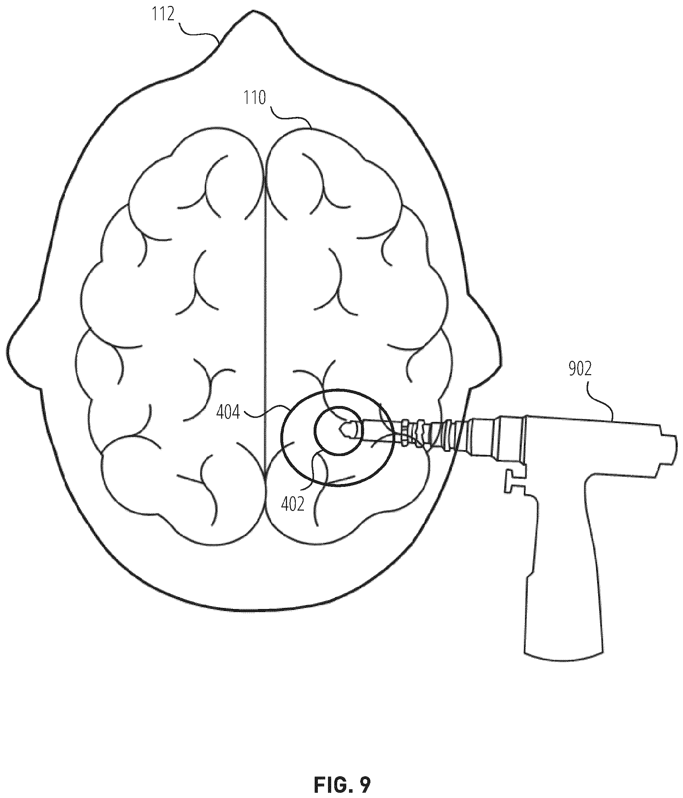

In summary, the method 800, including at least one of the operation 802 - operation 806, can contribute to a comprehensive approach that enhances the safety, efficiency, and effectiveness of the surgical procedure. The method 800, including operation 802 - operation 806, will be graphically represented and discussed herein with reference to FIG. 9.

FIG. 9 illustrates a schematic diagram showing an example three-dimensional model of a tool (e.g., three-dimensional model of tool 902) during a surgical procedure. As shown in FIG. 9, the projected structured light 110 can include the three-dimensional model of tool 902 along the planned entry point 402 and the safety zone 404 of the shape 220 (or the updated planned entry point 406 or the updated safety zone 408 of the adjusted shape 222 (all discussed in FIG. 4)) to show a relative orientation of the planned use of the three-dimensional model of tool 902 during the surgical procedure (or a step during the surgical procedure). As discussed with reference to the method 800 in FIG. 8, the three-dimensional model of tool 902 can also provide visualization of the tool in use, and showcase intraoperative adjustments made intraoperatively, which can help inform the doctor of the current best next step based on each previously completed procedures of the surgical procedure, enhanced precision in the use of the tool, and provide interactive guidance to the surgical team throughout the surgical procedure.

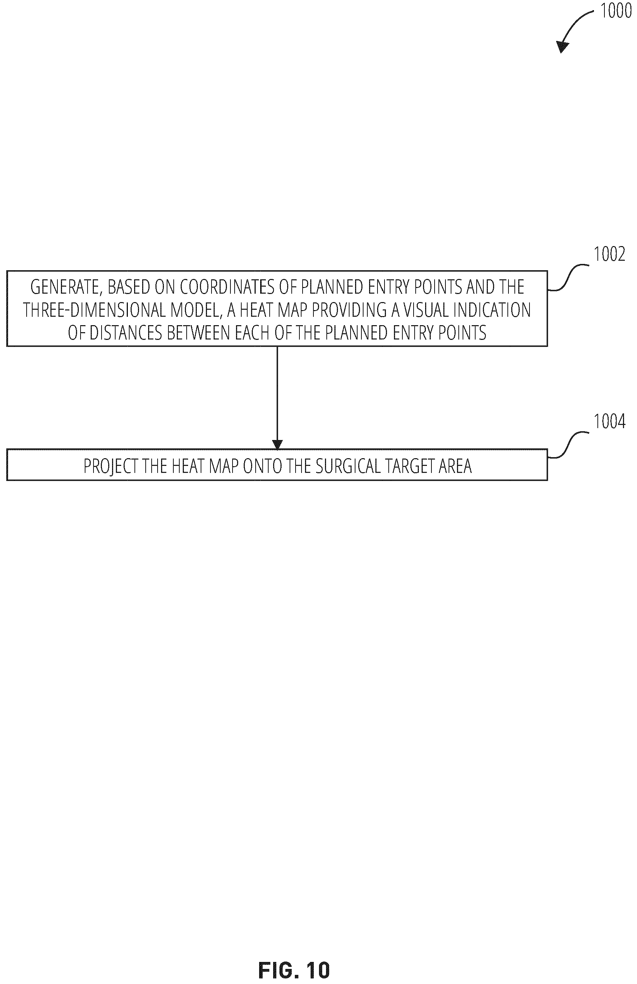

FIG. 10 illustrates a method 1000 for providing surgical guidance during a surgical procedure. Although the example method 1000 depicts a particular sequence of operations, the sequence may be altered. For example, some of the operations depicted may be performed in parallel or in a different sequence that does not materially affect the function of the method 1000. In other examples, different components of an example device or system that implements the method 1000 may perform functions at substantially the same time or in a specific sequence. The method 1000 can include at least one of operation 1002 or operation 1004.

In operation 1002, method 1000 can optionally include generating, based on coordinates of planned entry points (e.g., the planned entry point 402 (FIG. 4)) and the three-dimensional model (e.g., three-dimensional model 216 (FIG. 2)), a heat map. This heat map can be a visual tool that aids in the surgical planning and execution by providing a clear indication of the distances between each of the planned entry points. In examples, the heat map can be generated by integrating the coordinates of the planned entry points with the three-dimensional model of the patient's anatomy. The integration of the coordinates of the planned entry points and the three-dimensional model can ensure that the heat map accurately reflects the spatial relationships within the surgical target area. The heat map can enhance the spatial awareness of the surgical team throughout the surgical procedure. The heat map can also allow the surgical team to adjust the planned surgical procedure based on information shown by the heat map in the projected structured light 110.

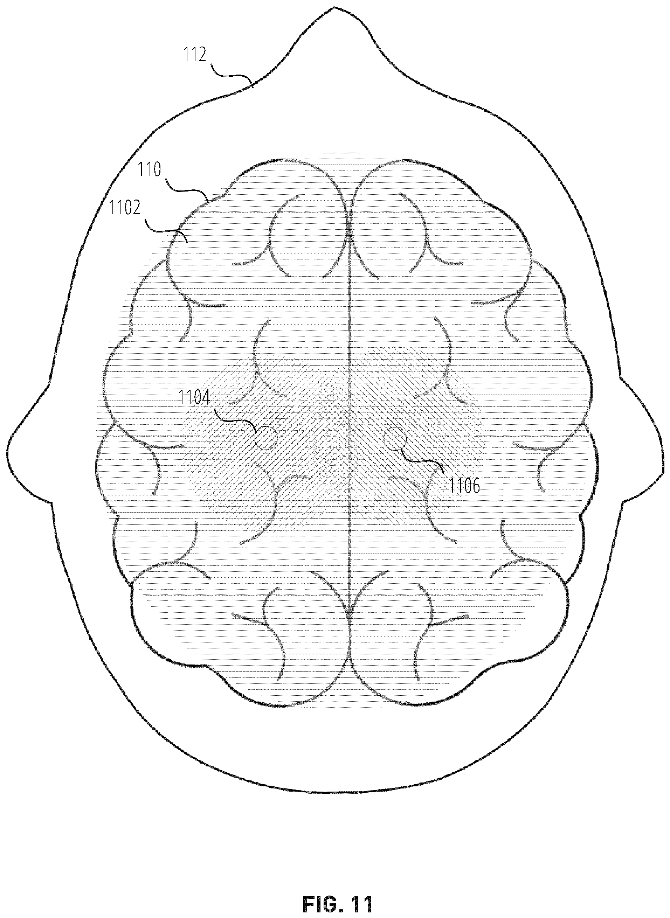

In operation 1004, method 1000 can optionally include projecting (e.g., via the projected structured light 110 (FIG. 1)) the heat map onto the surgical target area (e.g., target area for surgical procedure 114 (FIG. 1)). The heat map, generated in operation 1002, can serve as a visual guide to assist the surgical team in navigating the surgical target area with greater precision and awareness. For example, the projected heat map can provide a visual indication of the distances between planned entry points directly on the patient, allowing the surgical team to see how entry points relate to one another in real space. The projected heat map can guide the surgical time in real-time throughout the surgical procedure. Moreover, as adjustments are required during the surgical procedure, the heat map can be updated to reflect the updated plan based on the adjustments. The heat map will be shown in a graphical representation and discussed with reference to FIG. 11.

FIG. 11 illustrates an example heat map 1102 projected, via the projected structured light 110, onto a patient 112 during a surgical procedure. The heat map 1102 can be configured to enhance the surgical team's understanding of the spatial relationships between multiple entry points (e.g., a first planned entry point 1104 and a second planned entry point 1106). The heat map 1102 can thus improve the planning and execution of the surgical procedure. The heat map 1102 is a visual tool that is projected (e.g., via the projected structured light 110) onto the patient's body. The heat map 1102 can include color-coded, gradient representations, or other visual indicators of distances between various entry points (e.g., the first planned entry point 1104 and the second planned entry point 1106). These visual indicators provided by the heat map 1102 can help the surgical team to quickly assess the proximity of the entry points to one another.

The heat map 1102 provides the surgical team with a distance assessment, intraoperative reference, and dynamic interactions during the surgical procedure. The distance assessment can simply be the colors or gradient showing varying densities or intensities as two planned entry points are closer together. Such an indication can ensure the surgical team proceeds with caution to not interfere with future entry points or surgical points. The intraoperative reference can provide feedback for previous steps and guidance for the current step and future steps in the surgical procedure. The guidance can include real-time references, guiding incisions, or the placement of tools or instruments for the surgical team in real-time throughout the procedure. In examples, the surgical team can interact with the heat map to adjust one or more entry points or request different views of the heat map to improve the visibility of the projected structured light 110 or the patient themself. As such, the heat map 1102 can help improve the overall precision of the surgical procedure to potentially reduce the risk of complications and improve patient outcomes.

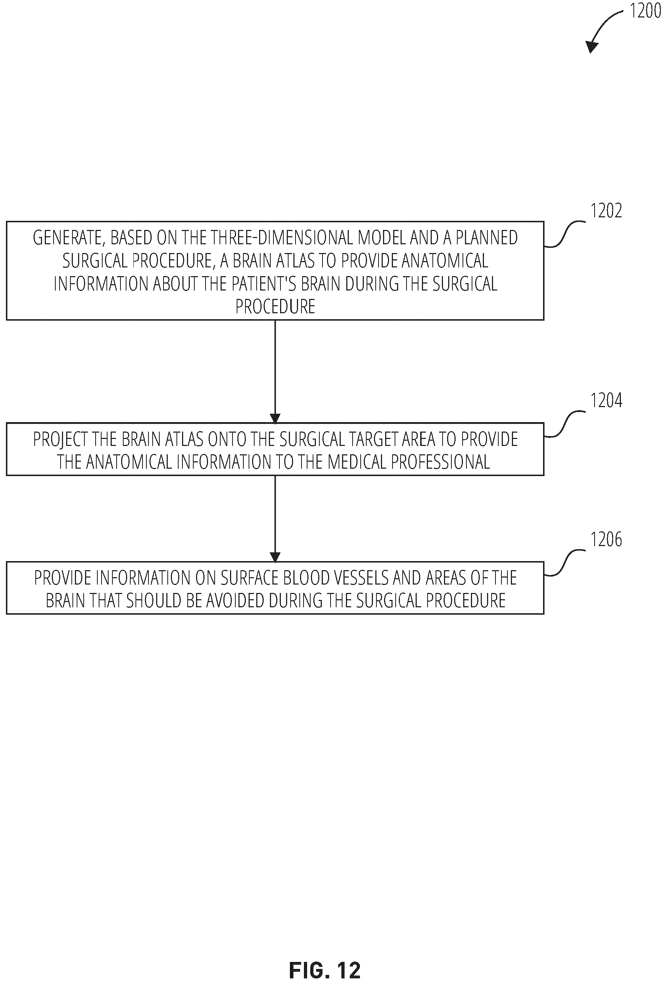

FIG. 12 illustrates a method 1200 for providing surgical guidance during a surgical procedure. Although the example method 1200 depicts a particular sequence of operations, the sequence may be altered . For example, some of the operations depicted may be performed in parallel or in a different sequence that does not materially affect the function of the method 1200. In other examples, different components of an example device or system that implements the method 1200 may perform functions at substantially the same time or in a specific sequence. The method 1200 can optionally include at least one of operation 1202 - operation 1206.

In operation 1202, method 1200 can optionally include generating, based on the three-dimensional model (e.g., three-dimensional (FIG. 2) and a planned surgical procedure, a brain atlas to provide anatomical information about the patient's brain during the surgical procedure. The brain atlas can be generated to include various anatomical features of the brain, such as gyri, sulci, blood vessels, and areas of interest specific to the planned surgical procedure. The brain atlas can aid the surgical team in identifying and navigating critical structures within the brain. The brain atlas can be tailored to the planned surgical procedure such that the brain atlas can provide relevant information to assist the surgical team during the surgical procedure. For example, if the surgery targets a tumor, the atlas can highlight the tumor location in relation to other brain structures.

In operation 1204, method 1200 can optionally include projecting the brain atlas onto the surgical target area to provide the anatomical information to the medical professional. The brain atlas can be projected as a part of the projected structured light 110 (FIG. 1). Though we are discussing a brain atlas herein, the projected structured light 110 can include any atlas of another anatomical region of a patient (e.g., heart, lungs, ankle, knee, hip, or any other anatomical portion of the patient).

In operation 1206, the method 1200 can optionally icnlude providing information on surface blood vessels and areas of the brain that should be avoided during the surgical procedure. As such, the brain map can include anatomical reference points that the surgical team will engage or interact with during the surgical procedure and anatomical regions or areas that the surgical team should avoid during the surgical procedure. The projected structured light 110 can also include additional information (e.g., such as labeling or other patient-specific information) for any of the anatomical areas within the brain atlas. As such, the medical professionals can engage with the system (e.g., system 100 (FIG. 1) to request more or different information be displayed by the brain atlas during the surgical procedure.

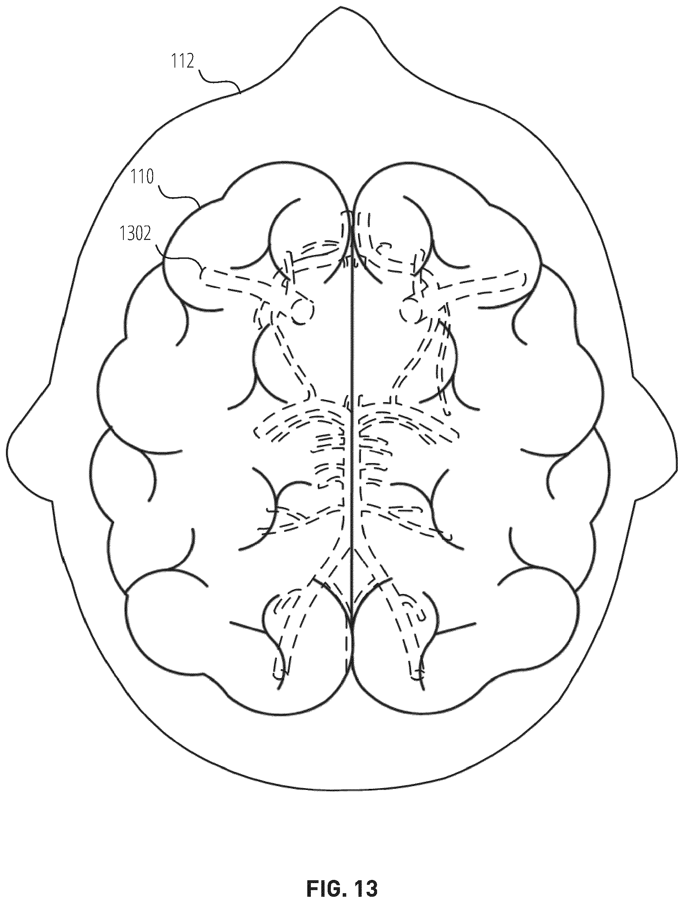

FIG. 13 illustrates an example brain atlas 1302 projected on a patient 112 during a surgical procedure. The brain atlas 1302 can be provided to surgical professionals via the projected structured light 110. As discussed herein, the brain atlas 1302 can provide anatomical information that is relevant to the surgical procedure. For example, anatomical information can be areas that will be engaged by the surgical team during the surgical procedure or areas that the surgical team should avoid during the surgical procedure. In examples, the engaged anatomical areas can be one color or shading, and the areas to avoid can be a different coloring or shading.

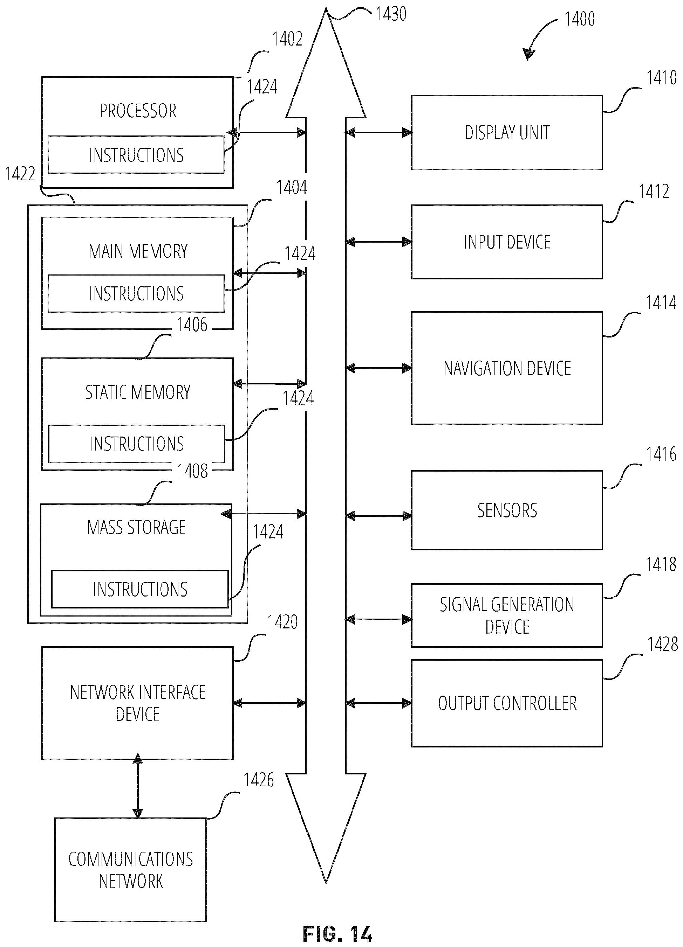

FIG. 14 illustrates a block diagram of an example machine 1400 upon which any one or more of the techniques (e.g., methodologies) discussed herein may perform. Examples, as described herein, may include, or may operate by, logic or a number of components, or mechanisms in the machine 1400. Circuitry (e.g., processing circuitry) is a collection of circuits implemented in tangible entities of the machine 1400 that include hardware (e.g., simple circuits, gates, logic, etc.). Circuitry membership may be flexible over time. Circuitries include members that may, alone or in combination, perform specified operations when operating. In an example, hardware of the circuitry may be immutably designed to carry out a specific operation (e.g., hardwired). In an example, the hardware of the circuitry may include variably connected physical components (e.g., execution units, transistors, simple circuits, etc.) including a machine readable medium physically modified (e.g., magnetically, electrically, moveable placement of invariant massed particles, etc.) to encode instructions of the specific operation. In connecting the physical components, the underlying electrical properties of a hardware constituent are changed, for example, from an insulator to a conductor or vice versa. The instructions enable embedded hardware (e.g., the execution units or a loading mechanism) to create members of the circuitry in hardware via the variable connections to carry out portions of the specific operation when in operation. Accordingly, in an example, the machine readable medium elements are part of the circuitry or are communicatively coupled to the other components of the circuitry when the device is operating. In an example, any of the physical components may be used in more than one member of more than one circuitry. For example, under operation, execution units may be used in a first circuit of a first circuitry at one point in time and reused by a second circuit in the first circuitry, or by a third circuit in a second circuitry at a different time. Additional examples of these components with respect to the machine 1400 follow.

In alternative examples, the machine 1400 may operate as a standalone device or may be connected (e.g., networked) to other machines. In a networked deployment, the machine 1400 may operate in the capacity of a server machine, a client machine, or both in server-client network environments. In an example, the machine 1400 may act as a peer machine in peer-to-peer (P2P) (or other distributed) network environment. The machine 1400 may be a personal computer (PC), a tablet PC, a set-top box (STB), a personal digital assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), other computer cluster configurations.

The machine 1400 may include a hardware processor 1402 (e.g., a central processing unit (CPU), a graphics processing unit (GPU), a hardware processor core, or any combination thereof), a main memory 1404, a static memory (e.g., memory or storage for firmware, microcode, a basic-input-output (BIOS), and mass storage 1408 (e.g., hard drives, tape drives, flash storage, or other block devices) some or all of which may communicate with each other via an interlink 1430 (e.g., bus). The machine 1400 may further include a display unit 1410, an alphanumeric input device 1412 (e.g., a keyboard), and a user interface (UI) navigation device 1414 (e.g., a mouse). In examples, the display unit 1410, input device 1412 and UI navigation device 1414 may be a touch screen display. The machine 1400 may additionally include a signal generation device 1418 (e.g., a speaker), a network interface device 1420, and one or more sensors 1416, such as a global positioning system (GPS) sensor, compass, accelerometer, or other sensor. The machine 1400 may include an output controller 1428, such as a serial (e.g., universal serial bus (USB), parallel, or other wired or wireless (e.g., infrared (IR), near field communication (NFC), etc.) connection to communicate or control one or more peripheral devices (e.g., a printer, card reader, etc.).

Registers of the processor 1402, the main memory 1404, the static memory 1406, or the mass storage 1408 may be, or include, a machine-readable medium 522 on which is stored one or more sets of data structures or instructions 1424 (e.g., software) embodying or utilized by any one or more of the techniques or functions described herein. The instructions 1424 may also reside, completely or at least partially, within any of registers of the processor 1402, the main memory 1404, the static memory 1406, or the mass storage 1408 during execution thereof by the machine 1400. In an example, one or any combination of the hardware processor 1402, the main memory 1404, the static memory 1406, or the mass storage 1408 may constitute the machine-readable media 1422. While the machine-readable medium 1422 is illustrated as a single medium, the term "machine-readable medium" may include a single medium or multiple media (e.g., a centralized or distributed database, and/or associated caches and servers) configured to store the one or more instructions 1424.

The term "machine-readable medium" may include any medium that is capable of storing, encoding, or carrying instructions for execution by the machine 1400 and that cause the machine 1400 to perform any one or more of the techniques of the present disclosure, or that is capable of storing, encoding or carrying data structures used by or associated with such instructions. Non-limiting machine readable medium examples may include solid-state memories, optical media, magnetic media, and signals (e.g., radio frequency signals, other photon-based signals, sound signals, etc.). In an example, a non-transitory machine-readable medium comprises a machine-readable medium with a plurality of particles having invariant (e.g., rest) mass, and thus are compositions of matter. Accordingly, non-transitory machine-readable media are machine-readable media that do not include transitory propagating signals. Specific examples of non-transitory machine-readable media may include: non-volatile memory, such as semiconductor memory devices (e.g., Electrically Programmable Read-Only Memory (EPROM), Electrically Erasable Programmable Read-Only Memory (EEPROM)) and flash memory devices; magnetic disks, such as internal hard disks and removable disks; magneto-optical disks; and CD-ROM and DVD-ROM disks.

In an example, information stored or otherwise provided on the machine-readable medium 1422 may be representative of the instructions 1424, such as instructions 1424 themselves or a format from which the instructions 1424 may be derived. This format from which the instructions 1424 may be derived may include source code, encoded instructions (e.g., in compressed or encrypted form), packaged instructions (e.g., split into multiple packages), or the like. The information representative of the instructions 1424 in the machine-readable medium 1422 may be processed by processing circuitry into the instructions to implement any of the operations discussed herein. For example, deriving the instructions 1424 from the information (e.g., processing by the processing circuitry) may include: compiling (e.g., from source code, object code, etc.), interpreting, loading, organizing (e.g., dynamically or statically linking), encoding, decoding, encrypting, unencrypting, packaging, unpackaging, or otherwise manipulating the information into the instructions 1424.

In an example, the derivation of the instructions 1424 may include assembly, compilation, or interpretation of the information (e.g., by the processing circuitry) to create the instructions 1424 from some intermediate or preprocessed format provided by the machine-readable medium 1422. The information, when provided in multiple parts, may be combined, unpacked, and modified to create the instructions 1424. For example, the information may be in multiple compressed source code packages (or object code, or binary executable code, etc.) on one or several remote servers. The source code packages may be encrypted when in transit over a network and decrypted, uncompressed, assembled (e.g., linked) if necessary, and compiled or interpreted (e.g., into a library, stand-alone executable etc.) at a local machine, and executed by the local machine.

The instructions 1424 may be further transmitted or received over a communications network 1426 using a transmission medium via the network interface device 1420 utilizing any one of a number of transfer protocols (e.g., frame relay, internet protocol (IP), transmission control protocol (TCP), user datagram protocol (UDP), hypertext transfer protocol (HTTP), etc.). Example communication networks may include a local area network (LAN), a wide area network (WAN), a packet data network (e.g., the Internet), LoRa/LoRaWAN, or satellite communication networks, mobile telephone networks (e.g., cellular networks such as those complying with 3G, 4G LTE/LTE-A, or 5G standards), Plain Old Telephone (POTS) networks, and wireless data networks (e.g., Institute of Electrical and Electronics Engineers (IEEE) 802.11 family of standards known as Wi-Fi^{®}, IEEE 802.15.4 family of standards, peer-to-peer (P2P) networks, among others. In an example, the network interface device 1420 may include one or more physical jacks (e.g., Ethernet, coaxial, or phone jacks) or one or more antennas to connect to the communications network 1426. In an example, the network interface device 1420 may include a plurality of antennas to wirelessly communicate using at least one of single-input multiple-output (SIMO), multiple-input multiple-output (MIMO), or multiple-input single-output (MISO) techniques. The term "transmission medium" shall be taken to include any intangible medium that is capable of storing, encoding or carrying instructions for execution by the machine 1400, and includes digital or analog communications signals or other intangible medium to facilitate communication of such software. A transmission medium is a machine-readable medium.

As discussed herein with reference to FIGS. 1-14, the systems, methods or apparatuses can be used for traditional surgical procedures as well as robotic surgical procedures. As such, the system can also be configured to communicate with a navigation system of a surgical robot, which can adjust the surgical procedure based on at least one output of the systems described in FIGS. 1-14.

The above-detailed description includes references to the accompanying drawings, which form a part of the detailed description. The drawings show, by way of illustration, specific examples that may be practiced. These embodiments are also referred to herein as "examples."

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In the appended claims, the terms "including" and "in which" are used as the plain-English equivalents of the respective terms "comprising" and "wherein." Also, in the following claims, the terms "including" and "comprising" are open-ended, that is, a system, device, article, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The term "about," as used herein, means approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10%. In one aspect, the term "about" means plus or minus 10% of the numerical value of the number with which it is being used. Therefore, about 50% means in the range of 45%-55%. Numerical ranges recited herein by endpoints include all numbers and fractions subsumed within that range (e.g., 1 to 5 includes 1, 1.5, 2, 2.75, 3, 3.90, 4, 4.24, and 5). Similarly, numerical ranges recited herein by endpoints include subranges subsumed within that range (e.g., 1 to 5 includes 1-1.5, 1.5-2, 2-2.75, 2.75-3, 3-3.90, 3.90-4, 4-4.24, 4.24-5, 2-5, 3-5, 1-4, and 2-4). It is also to be understood that all numbers and fractions thereof are presumed to be modified by the term "about."

The above description is intended to be illustrative, and not restrictive. The Abstract is to allow the reader to quickly ascertain the nature of the technical disclosure and is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description, with each claim standing on its own as a separate embodiment. The scope of the examples should be determined with reference to the appended claims.

## Claims

1. A system (100) for projecting surgical instructions upon a patient during a surgical procedure, the system comprising:
a three-dimensional camera (102) equipped with a structured light projector (108); and
a computation module (104) communicatively coupled to the three-dimensional camera, the computation module including a processor and a memory device, the memory device including instructions that, when executed by the processor, cause the system to:
generate, based on pre-operative medical imaging data, a shape indicative of a planned procedure of the surgical instructions;
adjust, based on a three-dimensional model of a target area for the surgical procedure, the shape to generate an adjusted shape;
generate, based on coordinates of planned entry points and the three-dimensional model, a heat map providing a visual indication of a distance between each of the planned entry points; and
project, with the structured light projector, the adjusted shape and the heat map onto the target area of the surgical procedure to provide step-by-step procedures to a medical professional completing the surgical procedure.

2. The system of claim 1, wherein the shape comprises a drill hole indicium, the drill hole indicium including:
a circle indicative of a planned entry point; and
a safety zone surrounding the planned entry point.

3. The system of claim 2, wherein the safety zone indicates areas within the target area that must be shaved before the surgical procedure.

4. The system of any of claims 1-3, wherein the instructions configure the system to:
obtain, based on the planned procedure, a tool model representative of a tool for the planned procedure; and
project the tool model onto the target area for the surgical procedure to verify clearances and access to planned entry points.

5. The system of claim 4, wherein the instructions configure the system to:
adjust, based on a three-dimensional model of a target area for the surgical procedure, the tool model to generate an adjusted tool model; and
project, with the structured light projector, the adjusted tool model onto the target area of the surgical procedure to verify clearances and access to planned entry points with the adjusted tool model.

6. The system of any of claims 1-5, wherein the instructions configure the system to:
capture, with the three-dimensional camera, a three-dimensional image of the patient;
generate, using the three-dimensional image and the pre-operative medical imaging data, a planned surgical trajectory, wherein the planned surgical trajectory is configured to adjust for variation in the target area detected in the three-dimensional model; and
project, with the structured light projector, the planned surgical trajectory onto the patient.

7. The system of claim 6, wherein the instructions configure the system to:
project, with the structured light projector, a reference point on to the patient;
align the three-dimensional image and the pre-operative medical imaging data by matching the reference point captured in the three-dimensional image with a coordinate point located on the pre-operative medical imaging data; and
verify the planned surgical trajectory by confirming the alignment of the reference point and the coordinate point.

8. The system of any of claims 1-7, wherein the instructions configure the system to:
generate, based on the three-dimensional model and a planned surgical procedure, a brain atlas to provide anatomical information about a brain of the patient during the surgical procedure; and
project, with the structured light projector, the brain atlas to provide the anatomical information of the brain to the medical professional during the surgical procedure.

9. The system of claim 8, wherein the brain atlas includes surface blood vessels and other areas of the brain that should be avoided during the surgical procedure.

10. The system of any of claims 8-9, wherein the surgical procedure includes a craniotomy, and wherein the anatomical information provides an outline for the medical professional to complete the craniotomy.

## Patentansprüche

1. System (100) zum Projizieren chirurgischer Anweisungen auf einen Patienten während eines chirurgischen Vorgehens, wobei das System Folgendes umfasst:
eine dreidimensionale Kamera (102), die mit einem Projektor (108) für strukturiertes Licht ausgestattet ist; und
ein Rechenmodul (104), das kommunikativ an die dreidimensionale Kamera gekoppelt ist, wobei das Rechenmodul einen Prozessor und eine Speichervorrichtung enthält, wobei die Speichervorrichtung Anweisungen enthält, die, wenn sie durch den Prozessor ausgeführt werden, das System zu Folgendem veranlassen:
Erzeugen, basierend auf präoperativen medizinischen Bildgebungsdaten, einer Form, die ein geplantes Vorgehen der chirurgischen Anweisungen angibt;
Anpassen, basierend auf einem dreidimensionalen Modell eines Zielbereichs für das chirurgische Vorgehen, der Form, um eine angepasste Form zu erzeugen;
Erzeugen, basierend auf Koordinaten geplanter Eintrittspunkte und dem dreidimensionalen Modell, einer Heatmap, die eine visuelle Angabe einer Entfernung zwischen jedem der geplanten Eintrittspunkte bereitstellt; und
Projizieren, mit dem Projektor für strukturiertes Licht, der angepassten Form und der Heatmap auf den Zielbereich des chirurgischen Vorgehens, um einer medizinischen Fachkraft, die das chirurgische Vorgehen vornimmt, Schritt-für-Schritt-Vorgehen bereitzustellen.

2. System nach Anspruch 1, wobei die Form eine Bohrlochmarkierung umfasst, wobei die Bohrlochmarkierung Folgendes enthält:
einen Kreis, der einen geplanten Eintrittspunkt angibt; und
eine Sicherheitszone, die den geplanten Eintrittspunkt umgibt.

3. System nach Anspruch 2, wobei die Sicherheitszone Bereiche innerhalb des Zielbereichs angibt, die vor dem chirurgischen Vorgehen rasiert werden müssen.

4. System nach einem der Ansprüche 1-3, wobei die Anweisungen das System zu Folgendem konfigurieren:
Erlangen, basierend auf dem geplanten Vorgehen, eines Werkzeugmodells, das ein Werkzeug für das geplante Vorgehen darstellt; und
Projizieren des Werkzeugmodells auf den Zielbereich für das chirurgische Vorgehen, um Abstände und Zugang zu geplanten Eintrittspunkten zu überprüfen.

5. System nach Anspruch 4, wobei die Anweisungen das System zu Folgendem konfigurieren:
Anpassen, basierend auf einem dreidimensionalen Modell eines Zielbereichs für das chirurgische Vorgehen, des Werkzeugmodells, um ein angepasstes Werkzeugmodell zu erzeugen; und
Projizieren, mit dem Projektor für strukturiertes Licht, des angepassten Werkzeugmodells auf den Zielbereich des chirurgischen Vorgehens, um Abstände und den Zugang zu geplanten Eintrittspunkten mit dem angepassten Werkzeugmodell zu überprüfen.

6. System nach einem der Ansprüche 1-5, wobei die Anweisungen das System zu Folgendem konfigurieren:
Erfassen eines dreidimensionalen Bildes des Patienten mit der dreidimensionalen Kamera;
Erzeugen, unter Verwendung des dreidimensionalen Bildes und der präoperativen medizinischen Bildgebungsdaten, einer geplanten chirurgischen Bewegungsbahn, wobei die geplante chirurgische Bewegungsbahn dazu konfiguriert ist, eine Variation, die in dem dreidimensionalen Modell erkannt wurde, anzupassen; und
Projizieren, mit dem Projektor für strukturiertes Licht, der geplanten chirurgischen Bewegungsbahn auf den Patienten.

7. System nach Anspruch 6, wobei die Anweisungen das System zu Folgendem konfigurieren:
Projizieren, mit dem Projektor für strukturiertes Licht, eines Referenzpunkts auf den Patienten;
Ausrichten des dreidimensionalen Bildes und der präoperativen medizinischen Bildgebungsdaten durch Abgleichen des in dem dreidimensionalen Bild erfassten Referenzpunkts mit einem Koordinatenpunkt, der sich auf den präoperativen medizinischen Bildgebungsdaten befindet; und
Überprüfen der geplanten chirurgischen Bewegungsbahn durch Bestätigen der Ausrichtung des Referenzpunkts und des Koordinatenpunkts.

8. System nach einem der Ansprüche 1-7, wobei die Anweisungen das System zu Folgendem konfigurieren:
Erzeugen, basierend auf dem dreidimensionalen Modell und einem geplanten chirurgischen Vorgehen, eines Gehirnatlas, um anatomische Informationen über ein Gehirn des Patienten während des chirurgischen Vorgehens bereitzustellen; und
Projizieren, mit dem Projektor für strukturiertes Licht, des Gehirnatlas, um der medizinischen Fachkraft während des chirurgischen Vorgehens die anatomischen Informationen des Gehirns bereitzustellen.

9. System nach Anspruch 8, wobei der Gehirnatlas Oberflächenblutgefäße und andere Bereiche des Gehirns enthält, die während des chirurgischen Vorgehens vermieden werden sollten.

10. System nach einem der Ansprüche 8-9, wobei das chirurgische Vorgehen eine Kraniotomie enthält und wobei die anatomischen Informationen einen Umriss für die medizinische Fachkraft bereitstellen, um die Kraniotomie vorzunehmen.

## Revendications

1. Système (100) permettant de projeter des instructions chirurgicales sur un patient au cours d'une intervention chirurgicale, le système comprenant :
une caméra tridimensionnelle (102) équipée d'un projecteur de lumière structurée (108) ; et
un module de calcul (104) couplé en communication à la caméra tridimensionnelle, le module de calcul comprenant un processeur et un dispositif de mémoire, le dispositif de mémoire comprenant des instructions qui, lorsqu'elles sont exécutées par le processeur, amènent le système à :
générer, sur la base de données d'imagerie médicale préopératoire, une forme indiquant une intervention prévue des instructions chirurgicales ;
ajuster la forme, sur la base d'un modèle tridimensionnel d'une zone cible pour l'intervention chirurgicale, afin de générer une forme ajustée ;
générer, sur la base de coordonnées de points d'entrée prévus et du modèle tridimensionnel, une carte thermique fournissant une indication visuelle d'une distance entre chacun des points d'entrée prévus ; et
projeter, au moyen du projecteur de lumière structurée, la forme ajustée et la carte thermique sur la zone cible de l'intervention chirurgicale afin de fournir des procédures étape par étape à un professionnel de santé réalisant l'intervention chirurgicale.

2. Système de la revendication 1, dans lequel la forme comprend un repère de trou de forage, le repère de trou de forage comprenant :
un cercle indiquant un point d'entrée prévu ; et
une zone de sécurité entourant le point d'entrée prévu.

3. Système de la revendication 2, dans lequel la zone de sécurité indique des zones à l'intérieur de la zone cible qui doivent être rasées avant l'intervention chirurgicale.

4. Système de l'une quelconque des revendications 1 à 3, dans lequel les instructions configurent le système pour :
obtenir, sur la base de l'intervention prévue, un modèle d'outil représentatif d'un outil pour l'intervention prévue ; et
projeter le modèle d'outil sur la zone cible de l'intervention chirurgicale afin de vérifier les dégagements et l'accès aux points d'entrée prévus.

5. Système de la revendication 4, dans lequel les instructions configurent le système pour :
ajuster le modèle d'outil, sur la base d'un modèle tridimensionnel d'une zone cible pour l'intervention chirurgicale, afin de générer un modèle d'outil ajusté ; et
projeter, au moyen du projecteur de lumière structurée, le modèle d'outil ajusté sur la zone cible de l'intervention chirurgicale afin de vérifier, à l'aide du modèle d'outil ajusté, les dégagements et l'accès aux points d'entrée prévus.

6. Système de l'une quelconque des revendications 1 à 5, dans lequel les instructions configurent le système pour :
capturer, au moyen de la caméra tridimensionnelle, une image tridimensionnelle du patient ;
générer, à l'aide de l'image tridimensionnelle et des données d'imagerie médicale préopératoire, une trajectoire chirurgicale prévue, dans lequel la trajectoire chirurgicale prévue est configurée pour s'adapter aux variations de la zone cible détectées dans le modèle tridimensionnel ; et
projeter, au moyen du projecteur de lumière structurée, la trajectoire chirurgicale prévue sur le patient.

7. Système de la revendication 6, dans lequel les instructions configurent le système pour :
projeter, au moyen du projecteur de lumière structurée, un point de référence sur le patient ;
aligner l'image tridimensionnelle et les données d'imagerie médicale préopératoire en faisant correspondre le point de référence capturé dans l'image tridimensionnelle avec un point de coordonnées situé sur les données d'imagerie médicale préopératoire ; et
vérifier la trajectoire chirurgicale prévue en confirmant l'alignement du point de référence et du point de coordonnées.

8. Système de l'une quelconque des revendications 1 à 7, dans lequel les instructions configurent le système pour :
générer, sur la base du modèle tridimensionnel et d'une intervention chirurgicale prévue, un atlas cérébral pour fournir des informations anatomiques sur le cerveau du patient pendant l'intervention chirurgicale ; et
projeter, au moyen du projecteur de lumière structurée, l'atlas cérébral afin de fournir au professionnel de santé les informations anatomiques du cerveau pendant l'intervention chirurgicale.

9. Système de la revendication 8, dans lequel l'atlas cérébral comprend des vaisseaux sanguins superficiels et d'autres zones du cerveau à éviter pendant l'intervention chirurgicale.

10. Système de l'une quelconque des revendications 8 et 9, dans lequel l'intervention chirurgicale comprend une craniotomie, et dans lequel les informations anatomiques fournissent un contour permettant au professionnel de santé de réaliser la craniotomie.
